# EUROPEAN PATENT SPECIFICATION

(11) **EP 1 616 015 B1**
(45) Date of publication and mention of the grant of the patent: **08.08.2007**
(21) Application number: 04759170.6
(22) Date of filing: 07.04.2004
(51) Int. Cl.: C12N 15/82, A01H 5/00

(54) **METHODS AND COMPOSITIONS TO INCREASE PLANT RESISTANCE TO STRESS**
VERFAHREN UND ZUSAMMENSETZUNGEN ZUR ERHÖHUNG DER RESISTENZ VON PFLANZEN GEGENÜBER STRESS
PROCEDES ET COMPOSITIONS AUGMENTANT LA RESISTANCE DES PLANTES AU STRESS

(30) Priority: 09.04.2003 US 461345 P
(43) Date of publication of application: 18.01.2006
(73) Proprietor: Purdue Research Foundation, West Lafayette, IN 47906 (US)
(72) Inventor: BRESSAN, Ray, A., West Lafayette, IN 47906 (US)
(74) Representative: Bentham, Andrew
(86) International application number: PCT/US2004/010599
(87) International publication number: WO 2004/092326

(56) References cited:
- EP-A2- 1 033 405
- WO-A1-01/32002
- DATABASE EMBL [Online] 29 December 1999 (1999-12-29), "Arabidopsis thaliana putative transcription factor (MYB8) mRNA, complete cds." XP002314994 retrieved from EBI accession no. EM_PRO:AF207991 Database accession no. AF207991 -& STRACKE R ET AL: "THE R2R3-MYB GENE FAMILY IN ARABIDOPSIS THALIANA" CURRENT OPINION IN PLANT BIOLOGY, QUADRANT SUBSCRIPTION SERVICES, GB, vol. 4, no. 5, 2001, pages 447-456, XP001058081 ISSN: 1369-5266
- KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156
- ABE HIROSHI ET AL: "Arabidopsis AtMYC2 (bHLH) and AtMYB2 (MYB) function as transcriptional activators in abscisic acid signaling." PLANT CELL, vol. 15, no. 1, January 2003 (2003-01), pages 63-78, XP002314993 ISSN: 1040-4651
- KRANZ H D ET AL: "TOWARDS FUNCTIONAL CHARACTERISATION OF THE MEMBERS OF THE R2R3-MYB GENE FAMILY FROM ARABIDOPSIS THALIANA" PLANT JOURNAL, BLACKWELL SCIENTIFIC PUBLICATIONS, OXFORD, GB, vol. 16, no. 2, 1998, pages 263-276, XP000929447 ISSN: 0960-7412

### SEQUENCE LISTING

<110> PURDUE RESEARCH FOUNDATION
<120> METHODS AND COMPOSITIONS TO INCREASE PLANT RESISTANCE TO STRESS
<130> 3220-74797
<140> PCT/US04/10599
   <141> 2004-04-07
<150> 60/461,345
   <151> 2003-04-09
<160> 10
<170> PatentIn Ver. 3.2
<210> 1
   <211> 211
   <212> PRT
   <213> Arabidopsis thaliana
<400> 1
   Met Gly Arg Ser Pro Cys Cys Glu Lys Ala His Met Asn Lys Gly Ala
   1               5                   10                  15
   Trp Thr Lys Glu Glu Asp Gln Arg Leu Ile Asp Tyr Ile Arg Asn His
               20                  25                  30
   Gly Glu Gly Ser Trp Arg Ser Leu Pro Lys Ser Val Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Asp Gly Glu Glu Gln Ile Ile Val Lys
   65                  70                  75                  80
   Leu His Ser Leu Phe Gly Asn Lys Trp Ser Leu Ile Ala Gly Lys Leu
                   85                  90                  95
   Pro Gly Arg Thr Asp Asn Glu Ile Lys Asn Tyr Trp Asn Thr His Ile
               100                 105                 110
   Lys Arg Lys Leu Leu Asn Arg Gly Ile Asp Pro Lys Thr His Gly Ser
           115                 120                 125
   Ile Ile Glu Pro Lys Thr Thr Ser Phe His Pro Arg Asn Glu Asp Leu
       130                 135                 140
   Lys Ser Thr Phe Pro Gly Ser Val Lys Leu Lys Met Glu Thr Ser Cys
   145                 150                 155                 160
   Asn Cys Ala Ser Thr Ser Gly Thr Thr Thr Asp Glu Asp Leu Arg Leu
                   165                 170                 175
   Ser Val Asp Cys Asp Tyr Arg Tyr Asp His Leu Asp Lys Glu Leu Asn
               180                 185                 190
   Leu Asp Leu Thr Leu Gly Tyr Ser Pro Thr Arg Phe Val Gly Val Gly
           195                 200                 205
   Ser Cys Tyr
       210
<210> 2
   <211> 639
   <212> DNA
   <213> Arabidopsis thaliana
<400> 2
   atgggaagat caccatgttg tgaaaaggct cacatgaaca aaggagcatg gactaaagaa 60
   gaagatcaac gtcttattga ttatatacgt aatcatggtg aaggctcttg gcgttctctt 120
   cctaaatccg ttgggttgtt gcgttgtgga aaaagttgta gattaagatg gattaattac 180
   cttcgtcctg atcttaaacg tggaaatttc actgatggtg aagagcaaat cattgtcaaa 240
   cttcatagtt tatttggcaa caaatggtct ttgattgctg ggaaattacc gggaagaacc 300
   gataatgaga ttaaaaatta ttggaacact catataaaaa ggaagcttct taaccgtggt 360
   attgacccaa aaactcacgg ttcgatcatc gagcctaaaa cgacatcgtt tcatccccga 420
   aatgaagatt tgaagtccac gtttcccggt tctgttaaac taaagatgga gacttcttgt 480
   gaaaactgtg cttctacgag cggtacgact acggacgagg atttacggtt aagtgttgat 540
   tgtgattaca gatatgatca tctggacaag gagctaaact tggacctaac actcgggtac 600
   agtccgactc gatttgtcgg ggtcggatct tgttactag                        639
<210> 3
   <211> 236
   <212> PRT
   <213> Arabidopsis thaliana
<400> 3
   Met Gly Arg Ser Pro Cys Cys Glu Lys Ala His Thr Asn Lys Gly Ala
   1               5                   10                  15
   Trp Thr Lys Glu Glu Asp Gln Arg Leu Val Asp Tyr Ile Arg Asn His
               20                  25                  30
   Gly Glu Gly Cys Trp Arg Ser Leu Pro Lys Ser Ala Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Asp Asp Glu Asp Gln Ile Ile Ile Lys
   65                  70                  75                  80
   Leu His Ser Leu Leu Gly Asn Lys Trp Ser Leu Ile Ala Gly Arg Leu
                   85                  90                  95
   Pro Gly Arg Thr Asp Asn Glu Ile Lys Asn Tyr Trp Asn Thr His Ile
               100                 105                 110
   Lys Arg Lys Leu Leu Ser His Gly Ile Asp Pro Gln Thr His Arg Gln
           115                 120                 125
   Ile Asn Glu Ser Lys Thr Val Ser Ser Gln Val Val Val Pro Ile Gln
       130                 135                 140
   Asn Asp Ala Val Glu Tyr Ser Phe Ser Asn Leu Ala Val Lys Pro Lys
   145                 150                 155                 160
   Thr Glu Asn Ser Ser Asp Asn Gly Ala Ser Thr Ser Gly Thr Thr Thr
                   165                 170                 175
   Asp Glu Asp Leu Arg Gln Asn Gly Glu Cys Tyr Tyr Ser Asp Asn Ser
               180                 185                 190
   Gly His Ile Lys Leu Asn Leu Asp Leu Thr Leu Gly Phe Gly Ser Trp
           195                 200                 205
   Ser Gly Arg Ile Val Gly Val Gly Ser Ser Ala Asp Ser Lys Pro Trp
       210                 215                 220
   Cys Asp Pro Val Met Glu Ala Arg Leu Ser Leu Leu
   225                 230                 235
<210> 4
   <211> 294
   <212> PRT
   <213> Gossypium hirsutum
<400> 4
   Met Gly Arg Ser Pro Cys Cys Glu Lys Ala His Thr Asn Lys Gly Ala
   1               5                   10                  15
   Trp Thr Lys Glu Glu Asp Gln Arg Leu Ile Asn Tyr Ile Arg Val His
               20                  25                  30
   Gly Glu Gly Cys Trp Arg Ser Leu Pro Lys Ala Ala Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Glu Glu Glu Asp Glu Leu Ile Ile Lys
   65                  70                  75                  80
   Leu His Ser Leu Leu Gly Asn Lys Trp Ser Leu Ile Ala Gly Arg Leu
                   85                  90                  95
   Pro Gly Arg Thr Asp Asn Glu Ile Lys Asn Tyr Trp Asn Thr His Ile
               100                 105                 110
   Lys Arg Lys Leu Ile Ser Arg Gly Ile Asp Pro Gln Thr His Arg Pro
           115                 120                 125
   Leu Asn Gln Thr Ala Asn Thr Asn Thr Val Thr Ala Pro Thr Glu Leu
       130                 135                 140
   Asp Phe Arg Asn Thr Pro Thr Ser Val Ser Lys Ser Ser Ser Ile Lys
   145                 150                 155                 160
   Asn Pro Ser Leu Asp Phe Asn Tyr Asn Glu Phe Gln Phe Lys Ser Asn
                   165                 170                 175
   Thr Asp Ser Leu Glu Glu Pro Asn Cys Thr Thr Ser Ser Gly Met Thr
               180                 185                 190
   Thr Asp Glu Glu Gln Gln Glu Gln Leu His Lys Gln Gln Gln Tyr Asp
           195                 200                 205
   Pro Ser Asn Gly Gln Asp Leu Asn Leu Glu Leu Ser Ile Gly Ile Val
       210                 215                 220
   Ser Ala Asp Ser Ser Arg Val Ser Ser Ala Asn Ser Ala Glu Ser Lys
   225                 230                 235                 240
   Pro Lys Val Asp Asn Asn Asn Phe Gln Phe Leu Glu Gln Ala Met Val
                   245                 250                 255
   Ala Lys Ala Val Cys Leu Cys Trp Gln Leu Gly Phe Gly Thr Ser Glu
               260                 265                 270
   Ile Cys Arg Asn Cys Gln Asn Ser Asn Ser Asn Gly Phe Tyr Ser Tyr
           275                 280                 285
   Cys Arg Pro Leu Asp Ser
       290
<210> 5
   <211> 239
   <212> PRT
   <213> Oryza sativa
<400> 5
   Met Gly Arg Ser Pro Cys Cys Glu Lys Ala His Thr Asn Lys Gly Ala
   1               5                   10                  15
   Trp Thr Lys Glu Glu Asp Gln Arg Leu Ile Ala Tyr Ile Arg Ala His
               20                  25                  30
   Gly Glu Gly Cys Trp Arg Ser Leu Pro Lys Ala Ala Gly Leu Leu Arg
           35                  40                  45
   Cys Gly Lys Ser Cys Arg Leu Arg Trp Met Asn Tyr Leu Arg Pro Asp
       50                  55                  60
   Leu Lys Arg Gly Asn Phe Thr Asp Asp Glu Asp Glu Leu Ile Ile Arg
   65                  70                  75                  80
   Leu His Ser Leu Leu Gly Asn Lys Trp Ser Leu Ile Ala Gly Gln Leu
                   85                  90                  95
   Pro Gly Arg Thr Asp Asn Glu Ile Lys Asn Tyr Trp Asn Thr His Ile
               100                 105                 110
   Lys Arg Lys Leu Leu Ala Arg Gly Ile Asp Pro Gln Thr His Arg Pro
           115                 120                 125
   Leu Leu Ser Gly Gly Asp Gly Ile Ala Ala Ser Asn Lys Arg His His
       130                 135                 140
   Arg Arg Arg Ile Pro Tyr Pro Ser Arg Arg Arg Arg Arg Pro Arg Arg
   145                 150                 155                 160
   Ser Ser Pro Cys Glu Ala Ala Ala Ala Ala Ala Pro Gly Arg Leu Leu
                   165                 170                 175
   Gly Arg Arg Leu Pro Gln Gln Gln Arg His Asn Glu His Gly Gly Ala
               180                 185                 190
   Ala Val Pro Arg Pro Gln Pro Arg Ala Leu Gly Arg Ala Asp Ala Glu
           195                 200                 205
   Leu Ala Ala Gly Gly Asp Ala His Gln Arg Ala Ala Gly Leu Pro Leu
       210                 215                 220
   Leu Pro Pro Arg Leu Pro Arg Arg Gly Gly Val Gln Leu Ser Gly
   225                 230                 235
<210> 6
   <211> 273
   <212> PRT
   <213> Lycopersicon esculentum
<400> 6
   Met Gly Arg Ser Pro Cys Cys Glu Lys Ala His Thr Asn Lys Gly Ala
   1               5                   10                  15
   Trp Thr Lys Glu Glu Asp Glu Arg Leu Ile Ser Tyr Ile Arg Ala His
               20                  25                  30
   Gly Glu Gly Cys Trp Arg Ser Leu Pro Lys Ala Ala Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Glu Glu Glu Asp Glu Leu Ile Ile Lys
   65                  70                  75                  80
   Leu His Ser Leu Leu Gly Asn Lys Trp Ser Leu Ile Ala Gly Arg Leu
                   85                  90                  95
   Pro Gly Arg Thr Asp Asn Glu Ile Lys Asn Tyr Trp Asn Thr His Ile
               100                 105                 110
   Arg Arg Lys Leu Leu Ser Arg Gly Ile Asp Pro Thr Thr His Arg Ser
           115                 120                 125
   Ile Asn Asp Pro Thr Thr Ile Pro Lys Val Thr Thr Ile Thr Phe Ala
       130                 135                 140
   Ala Ala His Glu Asn Ile Lys Asp Ile Asp Gln Gln Asp Glu Met Ile
   145                 150                 155                 160
   Asn Ile Lys Ala Glu Phe Val Glu Thr Ser Lys Glu Ser Asp Asn Asn
                   165                 170                 175
   Glu Ile Ile Gln Glu Lys Ser Ser Ser Cys Leu Pro Asp Leu Asn Leu
               180                 185                 190
   Glu Leu Arg Ile Ser Pro Pro His His Gln Gln Leu Asp His His Arg
           195                 200                 205
   His His Gln Arg Ser Ser Ser Leu Cys Phe Thr Cys Ser Leu Gly Ile
       210                 215                 220
   Gln Asn Ser Lys Asp Cys Ser Cys Gly Ser Glu Ser Asn Gly Asn Gly
   225                 230                 235                 240
   Trp Ser Asn Asn Met Val Ser Met Asn Ile Met Ala Gly Tyr Asp Phe
                   245                 250                 255
   Leu Gly Leu Lys Thr Asn Gly Leu Leu Asp Tyr Arg Thr Leu Glu Thr
               260                 265                 270
   Lys
<210> 7
   <211> 22
   <212> DNA
   <213> Artificial Sequence
<220>
   <223> Description of Artificial Sequence: Synthetic primer
<400> 7
   acaaatcgag tcggactgta cc 22
<210> 8
   <211> 22
   <212> DNA
   <213> Artificial Sequence
<220>
   <223> Description of Artificial Sequence: Synthetic primer
<400> 8
   tccatcggct tactctacgt cg 22
<210> 9
   <211> 32
   <212> DNA
   <213> Artificial Sequence
<220>
   <223> Description of Artificial Sequence: Synthetic primer
<400> 9
   actggagctc atgggaagat caccatgttg tg 32
<210> 10
   <211> 32
   <212> DNA
   <213> Artificial Sequence
<220>
   <223> Description of Artificial Sequence: Synthetic primer
<400> 10
   acgttctaga cacacgagct agtaacaaga tc 32

## Description

### BACKGROUND

Environmental factors such as drought, soil salinity, and unfavorable temperatures lead to plant stress and result in reduced plant productivity. Crop yield losses of major crops such as rice, wheat, and maize due to abiotic stresses represent a significant economic and political factor and contribute to food shortages in many countries. Stress tolerant plants need to be developed to overcome these limitations and increase plant productivity. Traditional breeding strategies that attempt to select for genes that increase stress resistance have met with limited success because stress tolerance traits have complex inheritance making improvements by plant breeding schemes slow and unpredictable.

Most plants have evolved strategies to protect themselves against adverse environmental conditions such as drought and unfavorable temperatures. For example, when plants are exposed to unfavorable high growing temperatures, normal protein synthesis is reduced and rapid synthesis of heat shock proteins begins. Similarly, low temperature acclimation in plants is associated with the synthesis of hydrophilic proteins which act as cryoprotectants. Many plants respond to drought and salinity stress by accumulating high levels of proteins believed to protect plant tissues from osmotic stress. However, if the severity and duration of these stress conditions are intense or persist for a long time, the deleterious effects on plant development, growth and yield of most crop plants are significant. Continuous exposure to drought and high salt causes major alterations in the plant metabolism. These metabolic perturbations ultimately lead to cell death and consequently yield losses.

A generic stress signal transduction pathway is initiated by signal perception, followed by the generation of second messengers (*e.g.*, inositol phosphates and reactive oxygen species). The second messengers can modulate intracellular Ca²⁺ levels, often triggering a protein phosphorylation cascade that targets proteins directly involved in cellular protection or transcription factors controlling specific sets of stress-regulated genes. The products of these genes may participate in the generation of regulatory molecules like plant hormones such as abscisic acid (ABA), ethylene, and salicylic acid. These regulatory molecules can, in turn, propogate a second round of signaling response.

A plant hormone abscisic acid (ABA) mediates a variety of physiological processes, including the response to drought and salt stress. ABA is produced under water deficit conditions, which causes stomata closure and tolerance to drought and salt stress.

Altering the expression of certain transcription factors can greatly influence plant stress tolerance. For example, overexpression of the transcription factor C-BOX BINDING FACTOR-1 (CBF1) results in plants with increased tolerance to cold stress. Inducible expression of the transcription factor DEHYDRATION-RESPONSIVE ELEMENT BINDING-1A (DREB1A) also results in improved tolerance to several stress conditions, including drought, salt, and cold.

Stress signal transduction requires the proper spatial and temporal coordination of all related signaling molecules. MYB (originally identified as a transforming determinant in avian myeloblastosis virus) genes comprise a large family of transcription regulators in eukaryotes and are involved in a variety of biological functions. A common feature of MYB proteins is the presence of a functional DNA-binding domain (MYB domain) that is conserved among animals, plants and yeasts. The MYB domain typically consists of one to three imperfect (not identical) repeats, designated R1, R2 and R3. Each repeat is about 50 amino acids long and contains three helices, with the second and third helix forming the helix-tum-helix (HTH) DNA binding structure. MYB proteins show little sequence similarity outside the MYB domain and so are classified into subfamilies based on the number of repeats in the domain.

In plants, some MYB genes are characterized, and over 100 members have been identified in *Arabidopsis.* In contrast to animals, most plant MYB genes belong to the R2R3-MYB subfamily. Plant MYB genes have been shown to be involved in the regulation of many aspects of plant development, hormone signaling and metabolism. The "classical" MYB factors, which are related to c-Myb (cellular proliferation), may be involved in the control of the cell cycle in animals, plants and other higher eukaryotes. Functions of MYB proteins in plants include regulation of secondary metabolism, control of cellular morphogenesis and regulation of meristem formation and the cell cycle.

### SUMMARY OF THE DISCLOSURE

Regulators of genes, especially transcription factors, involved in plant stress tolerance pathways are useful for engineering tolerance into plants because a single gene can activate a whole cascade of genes leading to the tolerant phenotype.

A method for improving response of a plant to stress includes the steps of:
(a) transferring a DNA molecule, whose nucleotide sequence encodes a polypeptide that is at least 90% identical to an amino acid sequence as in SEQ ID NO: 1 to the plant; and
(b) expressing the DNA molecule in the plant.
The DNA molecule includes a nucleotide sequence as in SEQ ID NO: 2, either stably integrated in the plant genome or transiently expressed. Other DNA molecules which also improve plant response to stress, may also be transferred to further improve response.

The stress may be cold, osmotic, drought, or abcisic acid.

A polypeptide is an *Arabidopsis thaliana* HOS10 transcription factor as in SEQ ID NO: 1. The plant is a monocot or a dicot.

A transgenic plant includes a recombinant nucleic acid encoding a HOS 10 polypeptide (SEQ ID NO: 1), wherein increased expression of the protein within the plant results in increased cold resistance to the plant. The plant may be a monocot or dicot.

A plant seed includes a recombinant nucleic acid molecule encoding a polypeptide having an amino acid sequence that is at least 90% identical to SEQ ID NO: 1.

An expression cassette includes a promoter functional in a plant cell operably linked to an isolated nucleic acid sequence encoding an HOS 10 polypeptide (SEQ ID NO:2), leading to an enhanced expression of the polypeptide in the plant cell results in increased cold resistance to the plant.

The promoter may be stress induced, *e.g.*, an ABA-inducible promoter, a turgor-inducible promoter, and an ethylene responsive promoter.

The promoter may be a viral coat protein promoter, a plant tissue-specific promoter, a monocot promoter, a ubiquitin promoter, a CaMV 35S promoter, a CaMV 19S promoter, a *nos* promoter, an Adh promoter, a sucrose synthase promoter, a tubulin promoter, a napin promoter, an actin promoter, a *cab* promoter, a PEP Case promoter, a 7S-alpha'-conglycinin promoter, an R gene complex promoter, a tomato E8 promoter, a patatin promoter, a mannopine synthase promoter, a soybean seed protein glycinin promoter, a soybean vegetative storage protein promoter, a bacteriophage SP6 promoter, a bacteriophage T3 promoter, a bacteriophage T7 promoter, a P_{tac} promoter, and a root-cell promoter.

A plant vector includes a recombinant nucleic acid encoding a HOS10 polypeptide (SEQ ID NO: 1), wherein an expression of the polypeptide in a plant results in increased cold resistance of the plant.

A host plant cell includes a recombinant nucleic acid encoding a HOS10 polypeptide (SEQ ID NO: 1). Expression of the protein in the plant cells results in increased cold resistance of the plant.

A plant vector includes a recombinant nucleic acid encoding a HOS10 polypeptide (SEQ ID NO: 1). Expression of the polypeptide in a plant results in increased salt resistance of the plant.

A host plant cell includes a recombinant nucleic acid encoding a HOS10 protein (SEQ ID NO: 1). Expression of the polypeptide in a plant cell results in increased salt resistance of the plant.

A model plant used in the study of stress tolerance is *Arabidopsis thaliana.* A genetic screen that included a chimeric *RD29A::LUC* transgene and low light video imaging technology was used to isolate some *Arabidopsis* stress-responsive mutants. The *RD29A* promoter was cold-, osmotic stress- and ABA-responsive. Bioluminescent *Arabidopsis* plants expressing the *RD29A::LUC* transgene were used to identify some *Arabidopsis* mutants with altered induction of stress-responsive genes by high-salinity, cold and ABA. These mutants exhibited altered expression of the *RD29A::LUC* gene at a constitutive (*cos*), high (*hos*), or low (*los*) level in response to various abiotic-stress or ABA treatments.

### Definitions

"promoter" refers to regions or sequences of a nucleic acid molecule located upstream and/or downstream from the start of transcription and which are involved in recognition and binding of RNA polymerase and other proteins to initiate transcription. A "plant promoter" is a promoter capable of initiating transcription in plant cells. Such a promoter can be derived from plant genes or from other organisms, such as viruses capable of infecting plant cells. A stress-inducible promoter refers to a plant promoter capable of initiating transcription from an operably linked heterologous sequence in response to an exogenous stress response such as, for example, salinity, drought, and cold. The promoters need not be full length to function.

"operably linked" refers to functional linkage between a promoter and another nucleic acid molecule, wherein the promoter sequence initiates transcription ofRNA corresponding to the other sequence.

"nucleic acid sequence" refers to an ordered set of deoxyribonucleotide or ribonucleotide bases forming a single or double-stranded molecule.

"plant" includes whole plants, shoot vegetative organs/structures (e.g. leaves, stems and tubers), roots, flowers and floral organs/structures (e.g. bracts, sepals, petals, stamens, carpels, anthers and ovules), seed (including embryo, endosperm, and seed coat) and fruit (the mature ovary), plant tissue (e.g. vascular tissue, ground tissue, and the like) and cells (*e.g.* guard cells, egg cells, trichomes and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous.

"recombinant" refers to a human manipulated polynucleotide or a copy or a complement of a human manipulated polynucleotide. For instance, a recombinant expression cassette comprising a promoter operably linked to another polynucleotide may include a promoter that is heterologous to the other polynucleotide as the result of human manipulation.

"gene" refers to chromosomal DNA, plasmid DNA, cDNA, synthetic DNA, or other DNA that encodes a peptide, polypeptide, protein, or RNA molecule, and regions flanking the coding sequence involved in the regulation of expression.

"sequence identity" or "identity" in the context of two nucleic acid or polypeptide sequences refer to the residues in the two sequences that are the same when aligned for maximum correspondence over a specified comparison window. When percentage of sequence identity is used in reference to proteins, it is recognized that residue positions which are not identical often differ by conservative amino acid substitutions, where amino acid residues are substituted for other amino acid residues with similar chemical properties (*e.g.*, charge or hydrophobicity) and therefore do not change the functional properties of the molecule. Sequences that differ by such conservative substitutions are said to have "sequence similarity" or "similarity".

As used herein, *hos10-1* (and *myb8-1)* refer to a mutant DNA molecule caused by T-DNA insertion. *hos10 (myb8)* refers to the corresponding genes. HOS10 (MYB8) refers to the polypeptide encoded by the *hos10 (atmyb)* gene. HOS10 is synonymous with and AtMYB8 or simply MYB8.

### BRIEF DESCRIPTION OF THE DRAWINGS

FIG. 1 shows that polypeptide HOS10 negatively controls *RD29A::LUC* expression in response to either low temperature, the phytohormone abcisic acid (ABA), or osmotic stress. WT is on the left half of a vertical separation, HOS10-1 on the right. Mutant HOS 10-1 plant cells express more luminescence. (A) shows comparative luminescence; (B) quantified luminescence.

FIG. 2 shows that *hos10-1* mutants are susceptible to freezing treatment: (A) shows relative plant growth; (B) statistical analyses of relative survival; (C) electrolyte leakage assay.

FIG. 3 *hos10-1* mutant seed germination is hypersensitive to ABA inhibition (left panel) and *hos10-1* mutant plants lose more water under water deficit conditions (right panel). Error bars represent standard deviation (n=5 for left panel; n=8 for the right panel).

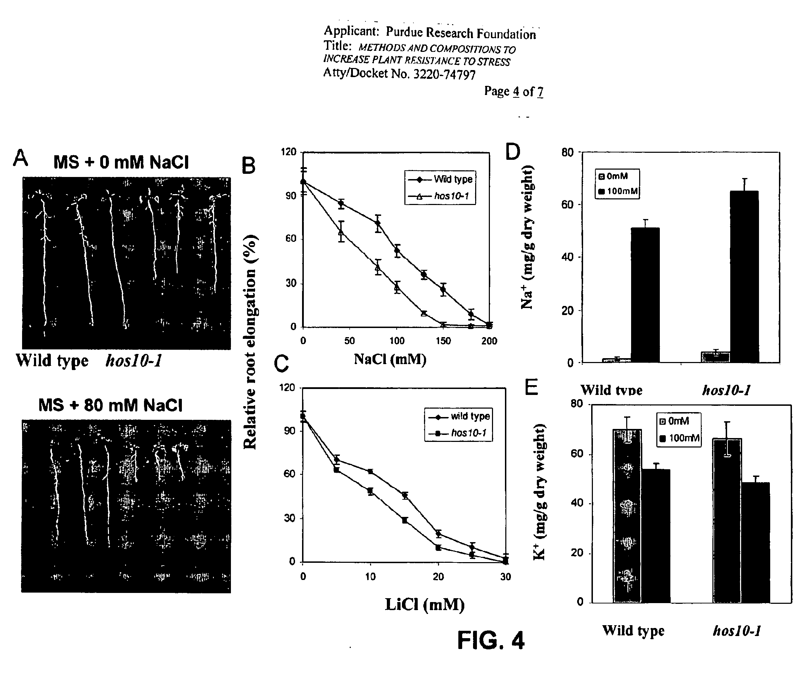

FIG. 4 shows that (A) and (B) *hos10-1* mutant plants are hypersensitive to NaCl, (C) LiCl; and (D) the mutant seedlings accumulated more Na⁺; (E) shows little difference in K accumulation.

FIG. 5 (A-E) shows developmental defects of *hos 10-1* plants. The right side of each photo are the mutants, on the left are the wild type plants.

FIG. 6 shows (A) the position of the of the insertion of the T-DNA at the *hos10-1* locus; (B) complementation of *hos10-1* mutant phenotype with the wild type *hos10* gene; and (C) sequence alignment comparing HOS10 (AtMYB8) in various plant species. HOS10 encodes a R2R3-type MYB transcription factor. The arrows represent the boundaries of the R2 and R3 domains.

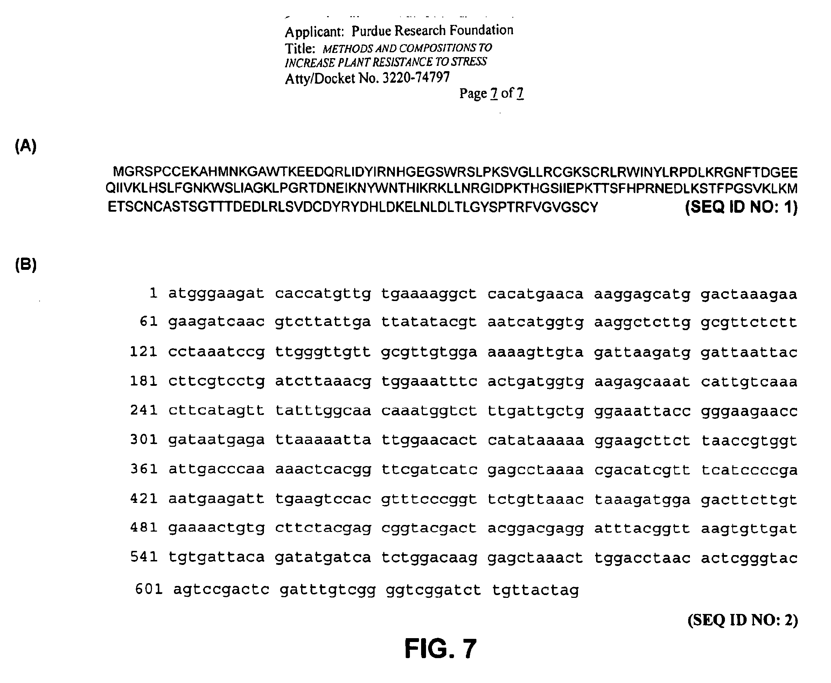

FIG. 7 shows the amino acid (SEQ ID NO: 1) (A) and the nucleotide (SEQ ID NO: 2) (B) sequence of the AtMYB8 transcription factor (HOS10).

### DETAILED DESCRIPTION OF THE DISCLOSURE

While the concepts of the present disclosure are illustrated and described in detail in the drawings and the description herein, such an illustration and description are to be considered as exemplary and not restrictive in character, it being understood that only the illustrative embodiments are shown and described.

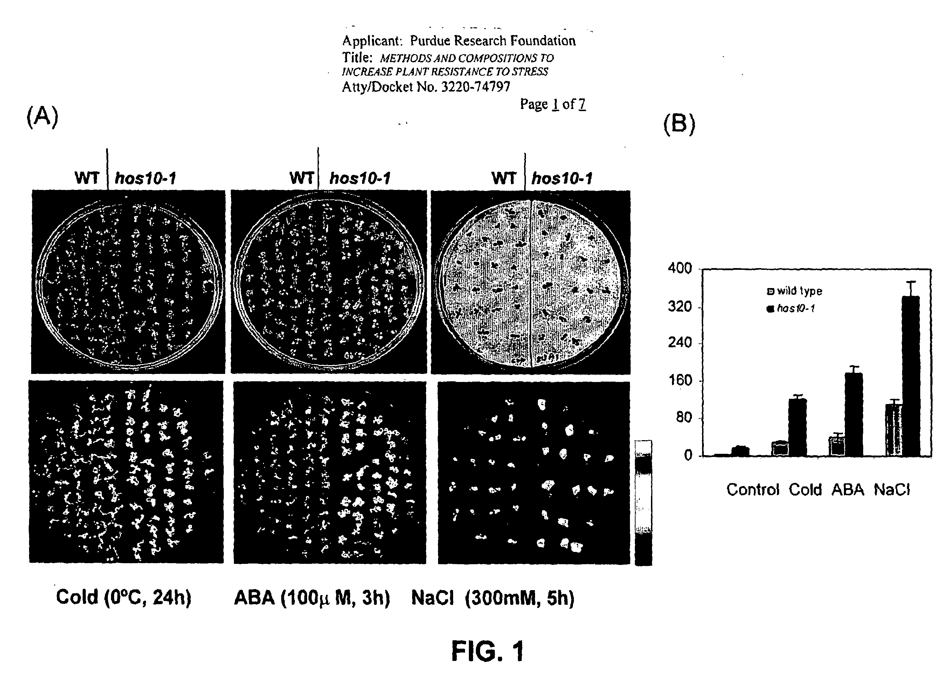

HOS10 appears to be a negative regulator of stress-responsive genes including *RD29A.* A mutant, *hos10-1,* which shows elevated induction of the *RD29A::LUC* gene in response to low temperature, ABA or NaCl (FIG. 1), was selected for characterization. The genetic locus responsible for this mutation was identified by TAIL-PCR, and *hos10* encodes a R2R3-type MYB transcription factor, corresponding to AtMYB8 (GenBank Accession number: AF207991; gene identifier At5g35515). Transgenic plants that express *hos10* either constitutively or in response to a stress response such as drought (*e.g.*, under an ABA-inducible promoter), show more resistance or tolerance towards the external stress than a plant that does not express *hos10,* or expresses it at a lower level.

FIG. 1 shows that *RD29A::LUC* expression is significantly higher in *hos10-1* than in the wild type seedlings in response to low temperature, ABA, and NaCl. The expression of *RD29A::LUC* is higher in *hos10-1* than wild type even under room temperature. The *hos10-1* mutation enhances the *RD29A::LUC* expression in response to cold, ABA or osmotic stress. (A) *RD29A::LUC* expression was quantitatively measured as luminescence intensity (counts/plant). Shown are wild type and *hos10-1* seedlings and their luminescence images taken at room temperature, or after treatment at 0°C, for 24h, or with 100 M ABA for 3h or 300 mM NaCl for 5h. The scale bar at the right shows the luminescence intensity from dark (lowest) to white (highest). (B) Quantification of luminescence images as shown in (A). Data represent the averages of luminescence intensities from 20 individual seedlings. Error bars indicate the standard deviation.

Because *hos10-1* mutant plants display enhanced sensitivity to cold, salinity, and ABA treatments, expression of HOS10 (MYB8) results in enhanced resistance or tolerance to these stress responses. For example, ectopic expression of HOS10 polypeptide in *hos10-1* mutant plants resulted in increased resistance to cold treatment compared to *hos10-1* mutant plants transformed with an empty vector (Example 1).

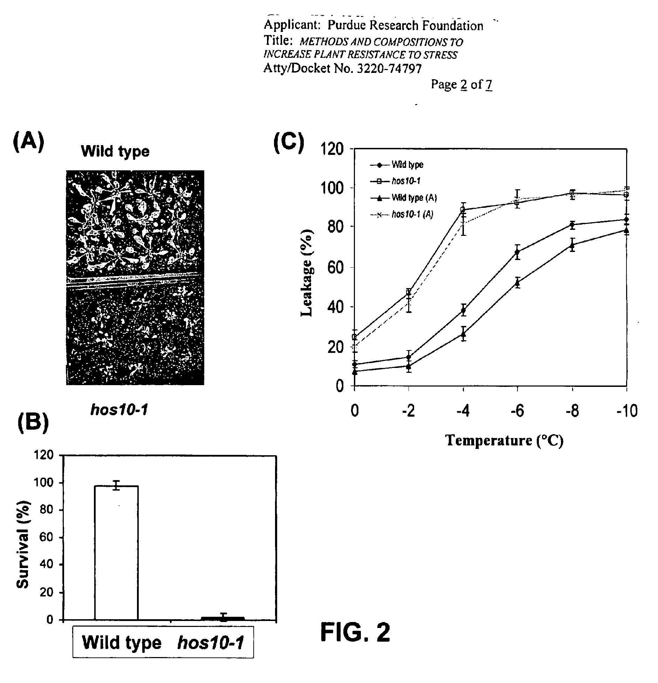

In freezing tolerance assays, *hos10-1* mutant plants appeared dramatically more sensitive to freezing (FIG. 2). *hos10-1* mutant plants were defective in cold acclimation (FIG. 2), leading to freezing sensitive phenotype. (A) Four-week-old wild type and *hos10-1* plants grown in a growth chamber were cold-acclimated at 4° C under light (16h light, 8h dark) for 8d before freezing treatment (-8° C for 4h). The photograph was taken 7d after the freezing treatment. (B) Statistical analysis of the plant survival 7d after the freezing treatment as shown in (A) The error bars indicate standard deviation (n=60). (C) Electrolyte leakage assay. Cold-induced acclimation was obtained by exposing plants at 4° C for 8d. *hos10-1* (A), *hos10-1* cold acclimated; wild type (A), wild type with cold acclimation. Error bars represent standard deviation (n=8)

Expression of *myb8 (hos10*) in a tissue specific manner is also within the scope of this disclosure. For example, expression of *myb8* (*hos10*) under a root-specific promoter results in the selective expression of *myb8 (hos10*) in plant roots and offers protection to root cells from high salinity or freezing. A root specific promoter includes for example a promoter for metallothionein-like gene PsMTA from pea (Fordam-Skelton, *et al.* 1997). Other examples of cis acting plant regulatory sequences for plant promoters can be found in http://rarge.gsc.riken.go.jp/cdna/promoter/ and Higo *et al.* (1999).

Tissue specific expression of *myb8 (hos10)* in flowers or any other floral parts results in increased tolerance to freezing. For example, expression under control of a petal or sepal specific promoter leads to freezing tolerance during adverse temperatures. A chalcone synthase (CHS) promoter from petunia is an example of a petal specific promoter.

Tissue specific expression of *myb8 (hos10)* in fruits, leaves, stems, petioles or any other plant part results in increased tolerance to freezing.

The observed higher induction of *RD29A* in the *hos10-1* mutant plants may be due to a feedback response due to hypersensitivity. Because the mutant is highly sensitive to stress treatments and stress-responsive genes such as *RD29A* are more easily activated to compensate for the reduced stress tolerance of the mutant. HOS10 is likely important both for stress tolerance and stress gene expression.

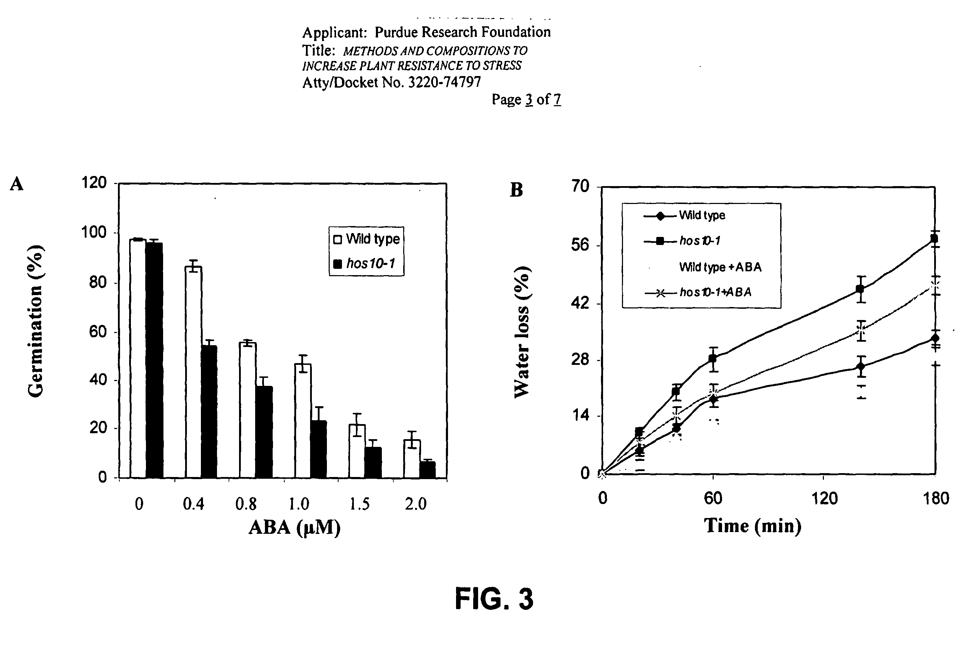

The *hos10-1* mutation appears to affect the ABA sensitivity and the water loss. (FIG. 3). *hos10-1* mutant seed germination is hypersensitive to ABA inhibition (left panel) and *hos10-1* mutant plants lose more water under water deficit conditions (right panel). Error bars represent standard deviation (n=5 for left panel; n=8 for the right panel).

The *hos 10-1* mutation appeared to affect the salt sensitivity of the mutant plants (FIG. 3). *hos 10-1* plants are hypersensitive to NaCl and LiCl as shown in (A) *hos 10-1* and wild type seedlings grown on MS medium or MS medium supplemented with 80 mM NaCl; (B) dosage response of *hos 10-1* and wild type seedlings to NaCl. Data shown are relative root growth (growth on 0 mM NaCl was considered 100%); (C) dosage response of *hos 10-1* and wild type seedlings to LiCl; Error bars in (B) and (C) indicate standard deviation (n=16). (D) Na+ accumulation in *hos 10-1* and wild type seedlings; Error bars indicate standard error (n=8); (E) K+ accumulation in *hos 10-1* and wild type seedlings. Error bars indicate standard error (n=8).

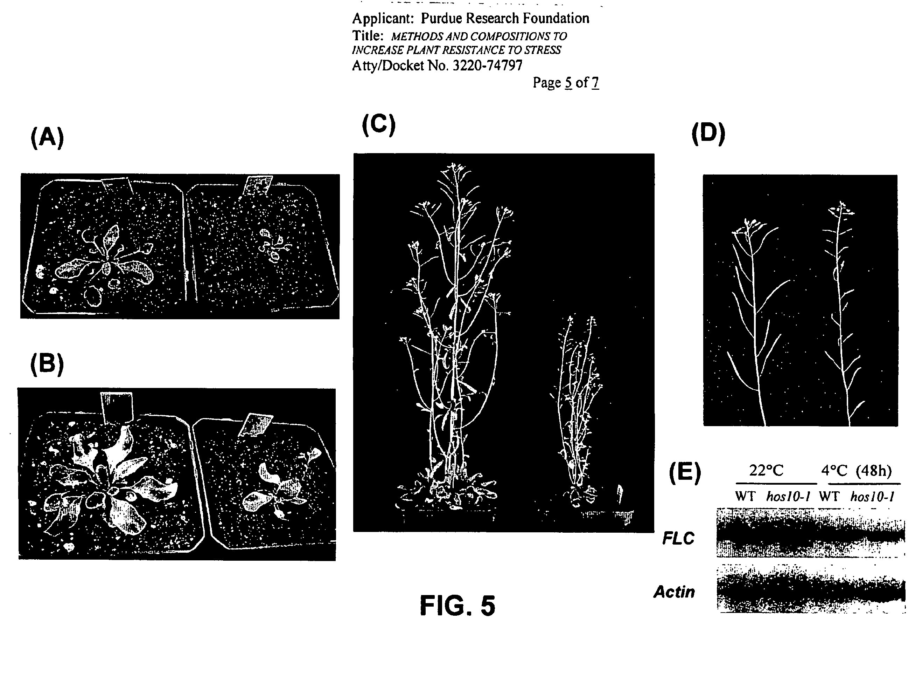

The *hos-10* locus was identified by TAIL-PCR and it encodes a R2R3-type MYB transcription factor. The wild type *atmyb8* gene was able to complement the *hos 10-1* mutant (FIG. 5).

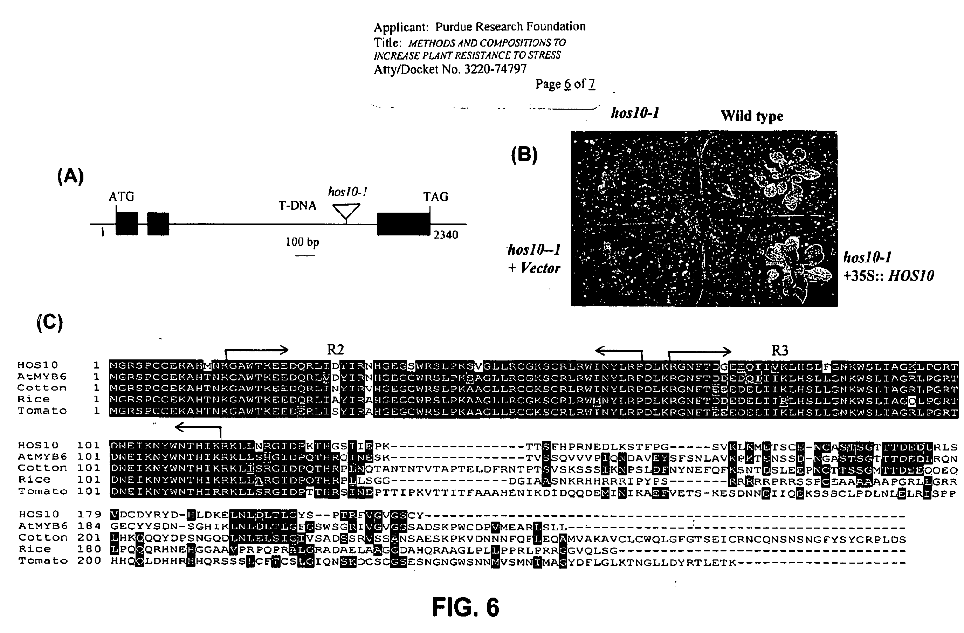

Expression of *atmyb8 (hos 10)* in *hos 10-1*/*myb8-1* mutant plants restored tolerance to freezing (FIG. 6). The structure of *hos10* gene and the position of the T-DNA insertion relative to the translation initiation codon which is the mutant form is shown in FIG. 6 (A). Filled boxes indicate exons; lines indicate introns. A T-DNA inserted in the *hos10-1*/*myb8-1* mutant is indicated; (B) shows that the genomic fragment containing the putative *hos10* gene complemented the *hos10-1*/*myb8-1* mutant. The photograph was taken 8d after the freezing treatment (-8C for 3h). *myb8-1*+vector, *myb8-1* transformed with the empty vector which was used for complementation); *myb8-1* +MYBB, *myb8-1* transformed with a wild type *atmyb8* gene); (C) sequence alignment comparing *hos10 (atmyb8)* with sequences from other species.

Depending upon the site of integration in the chromosome and the number of copies integrated, a transgenic plant may express the transgene to varying levels either constitutively or in response to a stimuli. Because the integration of a transgene is random, transgenic plants may have varying levels of expression. Standard RT-PCR or any other quantitative technique can be used to obtain a stably transformed transgenic plant selecting for desired expression levels.

Drought and low growth temperatures stresses may be governed by genes from multiple loci. For example, the cis-acting element identified in the promoter region of the RD29A gene, is responsive to both drought and low temperatures. This dehydration-responsive element (DRE) is required for the regulation of expression of dehydration responsive genes. CBF genes encode transcriptional activators that control the expression of a number of genes containing C-repeat/DRE sequences in their promoters. Other transcriptional regulators, such as the MYC and MYB proteins, are activators in the dehydration and abscisic acid (ABA)-inducible expression of the rd22 gene. The promoter of rd22 gene contains a 67-bp fragment with two closely situated recognition sites for the basic helix-loophelix protein MYC. There is also a putative recognition site for MYB in the promoter of the rd22 gene. All three recognition sites function as cis-acting elements in the dehydration-induced expression of rd22. Thus, it is contemplated to express a plurality of genes in plants, whose expression are regulated or triggered by a plurality of signaling pathways.

A suitable promoter is any one of the following; or any promoter that functions in a plant to allow expression of the *hos10* gene. A viral coat protein promoter, a tissue-specific promoter, a monocot promoter, a ubiquitin promoter, a CaMV 35S promoter, a CaMV 19S promoter, *a nos* promoter, an Adh promoter, a sucrose synthase promoter, a tubulin promoter, a napin promoter, an actin promoter, *a cab* promoter, a PEPCase promoter, a 7S-alpha'-conglycinin promoter, an R gene complex promoter, a tomato E8 promoter, a patatin promoter, a mannopine synthase promoter, a soybean seed protein glycinin promoter, a soybean vegetative storage protein promoter, a bacteriophage SP6 promoter, a bacteriophage T3 promoter, a bacteriophage T7 promoter, a P_{M,} promoter, a root-cell promoter, an ABA-inducible promoter or a turgor-inducible promoter, and any other stress inducible promoters. Stress inducible promoter are particularly helpful. Exemplary transiently activated stress-inducible plant promoters are described in U.S. Patent No. 6,414,221.

A plant vector includes an isolated nucleic acid encoding an HOS10 (AtMYB8) protein. Expression of the protein in a plant cell imparts increased cold, drought, and salt resistance to the plant cell. The plant vector can further include a promoter functional in a plant cell.

A transgenic plant includes an isolated nucleic acid (SEQ ID NO: 2) encoding an HOS 10 protein. Increased expression of the protein within a plant cell over its background level imparts increased cold, drought, and salt resistance to the plant cell.

Transgenic plants of the disclosure can be a dicot or a monocot. Examples of monocot plants are, maize, rice, rye, millet, wheat, barley, sorghum, oats, bananas, and orchids. Examples of dicot plants are *Arabidopsis,* cabbage, potato, tomato, tobacco, oranges and other citrus fruits, squashes, cotton, tea, soybeans and all the other legumes cashews, pistachios, apples, peaches, strawberries.

Signaling pathways are conserved in monocots and dicots. Several key signaling pathways are conserved among dicots and monocots. For example, mitogen activated protein kinase (MAPK) cascades in stress response, are conserved in monocots and dicots. In addition, MYB8 (HOS10) homologs from *Arabidopsis,* tomato, tobacco, and rice show significant structural similarity (FIG. 6C). Signaling mechanisms mediated by plant hormones such as abscisic acid (ABA) and ethylene are also conserved among plant species. Several genes from dicots such as *Arabidopsis* have been functionally expressed in monocots such as rice. Transgenic expression of MYB8 (HOS10) either constitutively or under an inducible or a tissue specific promoter results in increased resistance to stress tolerance in both monocots and dicots.

A seed includes an isolated nucleic acid encoding a HOS 10 polypeptide. Expression of the polypeptide within a plant cell imparts increased cold, drought, and salt resistance to the plant cell (SEQ ID NO: 1).

### EXAMPLES

### Example 1: Expression of HOS10 in hos10-1 mutant plants complements sensitivity to stress

The *hos10-1*/*myb8-1* mutant plants were transformed with an expression vector containing a wild-type *hos10* gene under the control of a CaMV 35S promoter using standard transformation techniques. FIG. 6 (B) shows that the genomic fragment containing the putative *hos10* gene complemented the *hos10-1*/*myb8-1* mutant. The photograph was taken 8d after the freezing treatment (-8C for 3h) The treatment samples were myb8-1+vector, *myb8-1* transformed with the empty vector (used for complementation); *myb8-1*+*myb8, myb8-1* transformed with a wild type *hos10* gene. The *hos10-1* mutant transformed with a wild-type *hos10* gene under the control of a CaMV 35S promoter were not susceptible to freezing conditions.

### Example 2: Overexpression of HOS10 Increases Plant Tolerance to Cold Temperature

Plants transformed with the vector containing only ³⁵S promoter (6), nontransformed (7) and ³⁵S promoter driving the *hos10* gene (1-5) were acclimated to cold (4°C) for 1 week. Plants were subsequently exposed to freezing temperatures 0°C to -16°C for 24 hr. The lowest temperature at which all of the plants survived was recorded and given as the cold tolerance of each transgenic line. Expression of the HOS10 gene was estimated relative to control (transformed with vector only) by RT-PCR of the HOS10 coding sequence cDNA.

**TABLE 1: Overexpression of HOS10 Increases Plant Tolerance to Cold Temperatures**

| Construct | Transgenic line No. | Expression of HOS10 | Cold tolerance phenotype (minimum temperature for survival °C) |
|---|---|---|---|
| ³⁵S promoter:HOS10 | 1 | ++++ | -14 |
| ³⁵S promoter:HOS 10 | 2 | +++ | -11 |
| ³⁵S promoter:HOS10 | 3 | +++ | -12 |
| ³⁵S promoter:HOS 10 | 4 | +++ | -12 |
| ³⁵S promoter:HOS10 | 5 | ++++ | -13 |
| ³⁵S promoter only | 6 | + | -9 |
| nontransformed | 7 | + | -9 |

The results of RT-PCR experiments (TABLE 1) show relative absorbance units of plants expressing HOS10 under a 35S promoter when compared to nontransformed plants. For example, absorbance units of a nontransformed plant (line no. 7; TABLE 1), was used as basal value (+) to calculate the relative expression of HOS10 in HOS 10 overexpressing plants.

### MATERIALS AND METHODS

### Transformation

A DNA segment identified in this disclosure is isolated, and combined with at least a promoter functional in a plant cell to provide a recombinant expression cassette. An expression cassette comprising a recombinant DNA segment is subcloned into any standard expression vector by methods known to those of skill in the art. Suitable expression vectors include plasmids that autonomously replicate in prokaryotic and/or eukaryotic cells. The expression vector is introduced into prokaryotic or eukaryotic cells by currently available methods such as, protoplast transformation, Agrobacterium-mediated transformation, electroporation, microprojectile bombardment, tungsten whiskers and liposomes. The vector is introduced into prokaryotic cells such as *E. coli* or *Agrobacterium.* Transformed cells is selected typically using a selectable or screenable marker encoded on the expression vector. A method to generate transgenic monocot plants is described in U.S. Patent No. 6,281,411, the disclosure of which is herein incorporated by reference as a method.

The expression cassette or vector is introduced into plant cells that include both monocot and dicot plants. Plant cells, or tissue, or organs useful for transformation include flowers, callus, immature embryos, meristematic tissue, gametic tissue, or cultured suspension cells. Other recombinant DNA molecules encoding proteins that enhance plant transformation may also be introduced. The transformed plant cell is regenerated into a plant and the plant tested for its ability to grow or thrive under stress conditions, such as high salinity, reduced water availability or adverse temperatures. Depending on the type of plant, the level of gene expression, and the activity of the protein encoded by the recombinant DNA segment, introduction of the recombinant DNA into the plant confers the phenotype of tolerance or resistance to the stress conditions to the plant.

The introduced recombinant DNA segments is expressed in the transformed plant cells and stably transmitted (somatically and sexually) to the subsequent cells produced. The vector is capable of introducing, maintaining, and expressing a recombinant DNA segment in plant cells, wherein the recombinant DNA is obtained from a variety of sources, including to plants and animals, bacteria, fungi, yeast or virus. Additionally, the vector is introduced into a wide variety of cells of plants.

Introduction and expression of foreign genes in dicotyledonous plants such as *Arabidopsis,* tobacco, potato and alfalfa has been accomplished using the T-DNA of the tumor-inducing (Ti) plasmid of *Agrobacterium tumefaciens.* Through recombinant DNA techniques, a wide variety of foreign DNAs can be inserted into the T-DNA in *Agrobacterium.* Following infection by the bacterium containing the recombinant Ti plasmid, the foreign DNA is inserted into the host plant chromosomes, thus producing a genetically engineered cell and then a genetically engineered plant. Another approach is to introduce root-inducing (Ri) plasmids as gene vectors. Additionally, using the Ti plasmid as a model system, it may be possible to artificially construct transformation vectors for monocot plants. Ti-plasmids may also be introduced into monocots by artificial methods such as microinjection, or fusion between monocot protoplasts and bacterial spheroplasts containing the T-region, which can then be integrated into the plant nuclear DNA. The selectable-marker and heterologous-gene expression cassettes described herein may be placed in a suitable expression vector designed for operation in plants. Suitable vectors are described in, for example, U.S. Pat. Nos. 5,888,789 and 5,889,189.

Transformation of plant cells with a recombinant DNA segment may also be accomplished by introducing a recombinant DNA into other nucleic acid molecules that can transfer the inserted DNA into a plant genome, *e.g.*, plant pathogens such as DNA viruses like CaMV or geminiviruses, RNA viruses, and viroids; DNA molecules derived from unstable plant genome components like extrachromosomal DNA elements in organelles (*e.g.*, chloroplasts or mitochondria), or nuclear encoded controlling elements; DNA molecules from stable plant genome components (*e.g.*, origins of replication and other DNA sequences which allow introduced DNA to integrate into the organellar or nuclear genomes and to replicate normally, to autonomously replicate, to segregate normally during cell division and sexual reproduction of the plant and to be inherited in succeeding generations of plants) or transposons.

A recombinant DNA may be delivered into plant cells or tissues directly by microorganisms with infectious plasmids, infectious viruses, the use of liposomes, microinjection by mechanical or laser beam methods, by whole chromosomes or chromosome fragments, electroporation, and microprojectile bombardment. For example, callus cells or any other suitable tissue derived from the members of a desired plant species are transformed with an expression cassette that includes at least the recombinant DNA segment of interest, a selectable marker, and a suitable promoter using a variety of standard techniques (*e.g.,* electroporation, *Agrobacterium,* protoplast fusion, or microparticle bombardment). A suitable transcription regulatory region (promoter) includes a plant promoter whose transcription is regulated in response to various stress responses such as drought, salinity, and high temperature.

**Isolation of *hos10-1* mutant**: *Arabidopsis thaliana* plants (ecotype C24) expressing the homozygous transgene *RD29A::LUC* referred to as wild type (Ishitani *et al.,* 1997) were mutagenized with an *Agrobacterium tumefaciens-mediated* (strain GV3101) T-DNA transformation using the activation tagging vector pSKI015 (Zhu *et al.,* 2002). Seeds from T₂ plants were used for screening mutants with altered *RD29A::LUC* expression in response to low temperature, ABA, and/or osmotic stress by luminescence imaging with a CCD camera as described by Ishitani *et al.* (1997).

**Freezing tolerance**: Wild type and *hos 10-1* plants grown in soil at the rosette stage were used for the freezing tolerance test. For cold acclimation treatment, plants were placed in a cold room at 4°C under a long day photoperiod (16h light, 8h dark). Fully developed rosette leaves were used to determine freezing-caused electrolyte leakage essentially as descried by Sukumaran and Weiser (1972) and Ristic and Ashworth (1993). Briefly, for each treatment, one excised leaflet was placed in a test tube containing 200 µl of HPLC grade H₂O, and the tubes were placed in a programmable cooling bath (model RTE-210; Thermo NesLab, Portsmouth, NH) with the temperature set at 0°C. There were 8 replicates for each temperature treatment. The temperature of the bath was programmed to decrease to -10°C, with 1°C decrement after 30 min. The tubes were removed from the bath when the designated temperature was reached, and they were placed immediately on ice water to allow gradual thawing for overnight. The tubes then were added with additional 5 ml HPLC H₂O and shaken for 24h, and the conductivity of the solution was measured with a conductance meter (model 35, YSI Co., Ohio). The tubes with the leaflets were then heated to boil for 40 min. After cooling down to room temperature, conductivities of the solutions were measured again. The percentage of electrolyte leakage was calculated as the percentage of the conductivity before boiling over that after boiling.

Three-week-old wild type and *hos10-1* plants grown in soil in a growth chamber were first incubated at 4°C under long day photoperiod for one week for cold acclimation. After this cold acclimation, the plants were subjected to freezing at -8°C for 4h. The plants were transferred immediately to 4°C after the treatment is finished and incubated overnight to allow the frozen soil to saw gradually. Plant damage was scored 7d later.

**Cloning of HOS10 gene:** DNA fragment flanking the left border of the inserted T-DNA in *hos10-1* plants was isolated by TAIL-PCR (Liu et al., 1995; Zhu et al., 2002) and subcloned into cloning vector pBluscript SK (+) (Stratagene, La Jolla, CA) as described (Zhu et al, 2002). The entire isolated fragment was sequenced. The following primer pair was designed to perform T-DNA diagnosis PCR (Zhu et al., 2002): forward 5'-ACAAATCGAGTCGGACTGTACC-3'; reverse, 5'-TCCATCGGCTTACTCTACGTCG-3'.

The coding region of HOS10 was amplified by *reverse transcription* (RT)-PCR. Total RNA was extracted using RNeasy Plant Mini Kit (Qiagen) from wild type plants (ecotype Columbia) and 3 µg of total RNA was used for first-strand cDNA synthesis using thermoscript^{™} RT-PCR system (Invitrogen, Carlsbad, CA). The gene specific primers for HOS10 were as follows: forward, 5'-ACTGGAGCTCATGGGAAGATCACCATGTTGTG-3' (SacI site is underlined) and reverse, 5'-ACGTTCTAGACACACGAGCTAGTAACAAGATC-3' (XbaI site is underlined). The RT-PCR product was then subcloned into pGEM-T Easy Vector with the pGEM-T Easy Vector System (Promega, Madison, WI), resulting the cDNA clonel0-133 and the sequence of the insert was confirmed by sequencing. The HOS10 was then released from clone 10-133 and cloned into binary vector 99-1 between SacI and XbaI sites, resulting the expression cassette of HOS10 under the CaM 35S promoter. The construct was introduced into *hos10-1* mutant plants through an *Agrobacterium tumefaciens*-mediated (strain GV3101) T-DNA transformation. Primary transformants, which were resistant to 50 mg/L hygromycin (Invitrogen), were transferred to soil to grow to maturity. Progenies of these transformants were examined for *RD29A::LUC* expression with the CCD camera and for freezing tolerance as described above.

**Ion content measurements and salt stress test**: Seeds were surface sterilized (2% sodium hypocloride for 15 min.), sowed onto medium containing Murashige and Skoog (MS) basal salt mixture, 2% sucrose solidified with 1% agar (pH 5.7). After sowing the seeds were stratified for 2 to 4 days at 4° C. For the mutant characterization *in vitro,* MS either contained no supplement or was supplemented with 40, 80, 100, 120, 150, 180, 200 mM NaCl, or 5, 10, 15, 20, 25, 30 mM LiCl. Three-week-old seedlings (n=40-60) grown on germination medium (0.7% agar) were transferred to a 500 ml flask containing 200 ml 1/2-strength MS salts, 2 % sucrose (pH 5.7). The flasks were shaken at 120 rpm under cool-fluorescent light (16h light, 8h dark) at 22°C. After 2d, NaCl was added to give the desired final NaCl concentrations, and the seedlings were allowed to grow for additional 2d. The seedlings were then harvested, rinsed with excess amount of deionized H₂O, dried at 65°C for 48h and weighed. Ground tissues (300 mg each) were then extracted with 0.1 M nitric acid for 4h and filtered through filter paper (Fisher Scientific). Na⁺ or K⁺ content was determined by atomic absorption spectrometer (Model SpectrAA-10, Varian, Springvale, Australia).

**Seed germination and water loss:** Surface-sterilized *hos10-1* and wild type seeds were sawn in Petri dishes on filter paper soaked with water or different concentrations of ABA. The Petri dishes were placed under light at room temperature for germination after being kept in darkness at 4°C for 3 days to break the dormancy. A seed was considered germinated when the radical emerged.

Detached shoots from soil-grown non-treated or ABA treated (100 µM ABA for 3h) *hos10-1* and wild type plants were placed on laboratory bench to lose water over a 3-hour time period. The water loss was expressed as the percentage of the original fresh weight.

**DOCUMENTS CITED**
Fordam-Skelton, et al. (1997) GUS expression in Arabidopsis directed by the 5' regions of the pea metallothionein-like gene PsMTA. Plant Molecular Biology 34: 659-668.
Higo et al. (1999) Plant cis-acting regulatory DNA elements (PLACE) database: 1999. Nucleic Acids Res, 27, 297-300.
Ishitani et al., (1997) Plant Cell 9 (11): 1935-49.
Liu et al., (1995). Efficient isolation and mapping of Arabidopsis thaliana T-DNA insert junctions by thermal asymmetric interlaced PCR. Plant J. 8,457-463.
Ristic and Ashworth (1993). Changes in leaf ultrastructure and carbohydrates in Arabidopsis thaliana L. (Heyn) cv. Columbia during rapid cold acclimation. Protoplasma 172: 111-123
Sukumaran and Weiser (1972) An excised leaflet test for evaluating potato frost tolerance. Hort. Science. 7: 467-468.
Zhu et al., (2002) Plant Cell 14(12):3009-28

## Claims

1. A method for improving response of a plant to stress, the method comprising:
(a) adding a DNA molecule, whose nucleotide sequence encodes a polypeptide that is at least 90% identical to an amino acid sequence as in SEQ ID NO: 1 to the plant
(b) expressing the DNA molecule in the plant.

2. The method of claim 1, wherein the DNA molecule comprises a nucleotide sequence as in SEQ ID NO: 2.

3. The method of claim 1, or 2 wherein the DNA molecule is stably integrated in the plant genome.

4. The method of claim 1, 2 or 3 wherein the stress is selected from the group consisting of cold, osmotic stress, drought, abcisic acid and salt.

5. The method of any one of the preceding claims wherein the polypeptide is an *Arabidopsis thaliana* HOS10 transcription factor as in SEQ ID NO: 1.

6. The method of any one of the preceding claims wherein the plant is a monocot.

7. The method of claim 6, wherein the monocot is maize, rice, rye, millet, wheat, barley, sorghum, banana, or an orchid.

8. The method of any one of claims 1 to 5 wherein the plant is a dicot.

9. The method of claim 8, wherein the dicot is Arabidopsis, cabbage, potato, tomato, tobacco, orange or another citrus fruit, squash, cotton, tea, soybean or another legume, cashew, pistachio, apple, peach or strawberry.

10. A method of any one of the preceding claims, wherein said DNA molecule is operably linked in an expression cassette to a promoter functional in a plant cell.

11. The method claim 10, wherein the promoter is stress induced.

12. The method of claim 11 wherein the stress induced promoter is selected from the group consisting of an ABA-inducible promoter, a turgor-inducible promoter, and an ethylene responsive promoter

13. The method of claim 12, wherein the promoter is selected from the group consisting of a viral coat protein promoter, a plant tissue-specific promoter, a monocot promoter, a ubiquitin promoter, a CaMV 35S promoter, a CaMV 19S promoter, a *nos* promoter, an Adh promoter, a sucrose synthase promoter, a tubulin promoter, a napin promoter, an actin promoter, a *cab* promoter, a PEP Case promoter, a 7S-alpha'-conglycinin promoter, an R gene complex promoter, a tomato E8 promoter, a patatin promoter, a mannopine synthase promoter, a soybean seed protein glycinin promoter, a soybean vegetative storage protein promoter, a bacteriophage SP6 promoter, a bacteriophage T3 promoter, a bacteriophage T7 promoter, a P_{tac} promoter, and a root-cell promoter.

14. A method of any one of the preceding claims, further comprising adding at least a second DNA molecule; and expressing the first and second DNA molecules in a plant.

15. The method of claim 14 wherein said second DNA molecule encodes a transcription factor in a different pathway than the one in which the DNA molecule of claim 1 is active.

16. The method of claim 14, wherein the expression of the first and second DNA molecules are controlled by different signalling pathways.

17. The method of claim 13 wherein the tissue-specific promoter is selected from the group consisting of root, flower, fruit, leaves, stem, and petiole specific promoters.

18. The method of any one of the preceding claims further comprising obtaining plant seed from said plant, said seed comprising said nucleic acid molecule encoding said polypeptide comprising said amino acid sequence at least 90% identical to SEQ ID NO: 1.

19. Use of a DNA molecule, whose nucleotide sequence encodes a polypeptide that is at least 90% identical to an amino acid sequence as in SEQ ID NO: 1, in the improvement of plant resistance to stress.

20. Use according to claim 19 wherein said stress is as defined in claim 4.

21. Use according to claim 19 wherein said plant is as defined in any one of claims 6 to 9.

## Patentansprüche

1. Verfahren zur Verbesserung der Reaktion einer Pflanze gegenüber Stress, worin das Verfahren
(a) Hinzufügen eines DNA-Moleküls, dessen Nukleotidsequenz ein Polypeptid codiert, das zumindest zu 90% identisch ist mit einer Aminosäuresequenz wie in SEQ ID NO:1, zu der Pflanze und
(b) Exprimieren des DNA-Moleküls in der Pflanze umfasst.

2. Verfahren nach Anspruch 1, worin das DNA-Molekül eine Nukleotidsequenz wie in SEQ ID NO:2 umfasst.

3. Verfahren nach Anspruch 1 oder 2, worin das DNA-Molekül stabil in das Pflanzengenom integriert ist.

4. Verfahren nach Anspruch 1, 2 oder 3, worin der Stress ausgewählt wird aus der Gruppe bestehend aus Kälte, osmotischem Stress, Trockenheit, Abscisinsäure und Salz.

5. Verfahren nach einem der vorhergehenden Ansprüche, worin das Polypeptid ein HOS10-Transkriptionsfaktor von *Arabidopsis thaliana* wie in SEQ ID NO:1 ist.

6. Verfahren nach einem der vorhergehenden Ansprüche, worin die Pflanze eine Monokotyledone ist.

7. Verfahren nach Anspruch 6, worin die Monokotyledone Mais, Reis, Roggen, Hirse, Weizen, Gerste, Sorghum, Banane oder eine Orchidee ist.

8. Verfahren nach einem der Ansprüche 1 bis 5, worin die Pflanze eine Dikotyledone ist.

9. Verfahren nach Anspruch 8, worin die Dikotyledone *Arabidopsis,* Kohl, Kartoffel, Tomate, Tabak, eine Orange oder eine andere Zitrusfrucht, Kürbis, Baumwolle, Tee, Sojabohne oder eine andere Hülsenfrucht, Acajoubaum, Pistazie, Apfel, Pfirsich oder Erdbeere ist.

10. Verfahren nach einem der vorhergehenden Ansprüche, worin das DNA-Molekül in einer Expressionskassette operativ mit einem in einer Pflanzenzelle funktionsfähigen Promotor verbunden ist.

11. Verfahren nach Anspruch 10, worin der Promotor stressinduziert ist.

12. Verfahren nach Anspruch 11, worin der stressinduzierte Promotor ausgewählt wird aus der Gruppe bestehend aus einem durch ABA induzierbaren Promotor, einem durch den Turgor induzierbaren Promotor und einem auf Ethylen ansprechenden Promotor.

13. Verfahren nach Anspruch 12, worin der Promotor ausgewählt wird aus der Gruppe bestehend aus einem Virushüllprotein-Promotor, einem pflanzengewebespezifischen Promotor, einem Monokotyledonen-Promotor, einem Ubiquitin-Promotor, einem CaMV 35S-Promotor, einem CaMV 19S-Promotor, einem nos-Promotor, einem Adh-Promotor, einem Saccharose-Synthase-Promotor, einem Tubulin-Promotor, einem Napin-Promotor, einem Actin-Promotor, einem cab-Promotor, einem PEPCase-Promotor, einem 7S-alpha'-Conglycinin-Promotor, einem R-Gen-Komplex-Promotor, einem Tomaten-E8-Promotor, einem Patatin-Promotor, einem Mannopin-Synthase-Promotor, einem Sojabohnensamenprotein-Glycinin-Promotor, einem Promotor eines vegetativen Speicherproteins der Sojabohne, einem Bakteriophagen-SP6-Promotor; einem Bakteriophagen-T3-Promotor, einem Bakteriophagen-T7-Promotor, einem P_{tac}-Promotor und einem Wurzelzell-Promotor.

14. Verfahren nach einem der vorhergehenden Ansprüche, weiter umfassend Hinzufügen zumindest eines zweiten DNA-Moleküls und Exprimieren des ersten und zweiten DNA-Moleküls in einer Pflanze.

15. Verfahren nach Anspruch 14, worin das zweite DNA-Molekül einen Transkriptionsfaktor in einem anderen Stoffwechselweg codiert als dem Stoffwechselweg, in welchem das DNA-Molekül nach Anspruch 1 aktiv ist.

16. Verfahren nach Anspruch 14, worin die Expression des ersten und zweiten DNA-Moleküls durch unterschiedliche Signalwege geregelt wird.

17. Verfahren nach Anspruch 13, worin der gewebespezifische Promotor ausgewählt wird aus der Gruppe bestehend aus wurzefspezifischen, blütenspezifischen, früchtespezifischen, blattspezifischen, stammspezifischen und blattstielspezifischen Promotoren.

18. Verfahren nach einem der vorhergehenden Ansprüche, weiter umfassend Erhalten von Pflanzensaatgut der Pflanze, wobei das Saatgut das Nukleinsäuremolekül umfasst, welches das Polypeptid codiert, welches die Aminosäuresequenz umfasst, die zumindest zu 90% mit SEQ ID NO:1 identisch ist.

19. Verwendung eines DNA-Moleküls, dessen Nukleotidsequenz ein Polypeptid codiert, welches zumindest zu 90% mit einer Aminosäuresequenz wie in SEQ ID NO:1 identisch ist, zu der Verbesserung der Resistenz einer Pflanze gegenüber Stress.

20. Verwendung nach Anspruch 19, worin der Stress wie in Anspruch 4 definiert ist.

21. Verwendung nach Anspruch 19, worin die Pflanze wie in einem der Ansprüche 6 bis 9 definiert ist.

## Revendications

1. Procédé pour améliorer la réponse d'une plante face à un stress, le procédé comprenant :
(a) l'addition à la plante d'une molécule d'ADN dont la séquence de nucléotides code pour un polypeptide qui est au moins à 90 % identique à une séquence d'acides aminés comme dans SEQ ID N0: 1 ; et
(b) l'expression de la molécule d'ADN dans la plante.

2. Procédé selon la revendication 1, dans lequel la molécule d'ADN comprend une séquence de nucléotides comme dans SEQ ID NO: 2.

3. Procédé selon la revendication 1 ou 2, dans lequel la molécule d'ADN est intégrée de façon stable dans le génome de la plante.

4. Procédé selon la revendication 1, 2 ou 3, dans lequel le stress est choisi dans le groupe constitué par le froid, un stress osmotique, la sécheresse, l'acide abscissique et le sel.

5. Procédé selon l'une quelconque des revendications précédentes, dans lequel le polypeptide est un facteur transcriptionnel HOS10 d'*Arabidopsis thaliana* comme dans SEQ ID NO: 1.

6. Procédé selon l'une quelconque des revendications précédentes, dans lequel la plante est une monocotylédone.

7. Procédé selon la revendication 6, dans lequel la monocotylédone est le maïs, le riz, le seigle, le millet, le blé, l'orge, le sorgho, la banane ou une orchidée.

8. Procédé selon l'une quelconque des revendications 1 à 5, dans lequel la plante est une dicotylédone.

9. Procédé selon la revendication 8, dans lequel la dicotylédone est Arabidopsis, le chou, la pomme de terre, la tomate, le tabac, l'orange ou un autre agrume, la courge, le coton, le thé, le soja ou une autre légumineuse, la noix de cajou, la pistache, la pomme, la pêche ou la fraise.

10. Procédé selon l'une quelconque des revendications précédentes, dans lequel ladite molécule d'ADN est liée de manière exploitable dans une cassette d'expression à un promoteur fonctionnel dans une cellule végétale.

11. Procédé selon la revendication 10, dans lequel le promoteur est induit par le stress.

12. Procédé selon la revendication 11, dans lequel le promoteur induit par le stress est choisi dans le groupe constitué par un promoteur inductible par l'ABA, un promoteur inductible par turgescence et un promoteur sensible à l'éthylène.

13. Procédé selon la revendication 12, dans lequel le promoteur est choisi dans le groupe constitué par un promoteur de protéine d'enveloppe virale, un promoteur spécifique au tissu végétal, un promoteur de monocotylédone, un promoteur de l'ubiquitine, un promoteur CaMV 35S, un promoteur CaMV 19S, un promoteur *nos,* un promoteur Adh, un promoteur de la saccharose synthase, un promoteur de la tubuline, un promoteur de la napine, un promoteur de l'actine, un promoteur *cab*, un promoteur de la PEPCase, un promoteur de la 7S-alpha'-conglycinine, un promoteur complexe du gène R, un promoteur E8 de la tomate, un promoteur de la patatine, un promoteur de la mannopine-synthase, un promoteur de la protéine-glycinine de la graine de soja, un promoteur de la protéine de réserve végétative du soja, un promoteur du bactériophage SP6, un promoteur du bactériophage T3, un promoteur du bactériophage T7, un promoteur P_{tac} et un promoteur de cellules de racines.

14. Procédé selon l'une quelconque des revendications précédentes, comprenant en outre l'addition d'au moins une seconde molécule d'ADN et l'expression des première et seconde molécules d'ADN dans une plante.

15. Procédé selon la revendication 14, dans lequel ladite seconde molécule d'ADN code pour un facteur transcriptionnel dans une voie différente de celle dans laquelle la molécule d'ADN selon la revendication 1 est active.

16. Procédé selon la revendication 14, dans lequel l'expression des première et seconde molécules d'ADN est contrôlée par différentes voies de signalisation.

17. Procédé selon la revendication 13, dans lequel le promoteur spécifique aux tissus est choisi dans le groupe constitué par les promoteurs spécifiques à la racine, à la fleur, au fruit, aux feuilles, à la tige et au pétiole.

18. Procédé selon l'une quelconque des revendications précédentes, comprenant en outre l'obtention d'une graine provenant de ladite plante, ladite graine comprenant ladite molécule d'acide nucléique codant pour ledit polypeptide comprenant ladite séquence d'acides aminés au moins à 90 % identique à SEQ ID N0: 1.

19. Utilisation d'une molécule d'ADN dont la séquence de nucléotides code pour un polypeptide qui est au moins à 90 % identique à une séquence d'acides aminés comme dans SEQ ID N0: 1, dans l'amélioration de la résistance de la plante face au stress.

20. Utilisation selon la revendication 19, dans laquelle ledit stress est tel que défini dans la revendication 4.

21. Utilisation selon la revendication 19, dans laquelle ladite plante est telle que définie dans l'une quelconque des revendications 6 à 9.
